# EUROPEAN PATENT SPECIFICATION

(11) **EP 1 919 397 B1**
(45) Date of publication and mention of the grant of the patent: **02.01.2013**
(21) Application number: 06787577.3
(22) Date of filing: 13.07.2006
(51) Int. Cl.: A61F 2/24

(54) **TWO-PIECE PERCUTANEOUS PROSTHETIC HEART VALVES**
ZWEITEILIGE PERKUTANE HERZKLAPPENPROTHESEN
VALVES CARDIAQUES PROTHETIQUES PERCUTANEES PRESENTANT DEUX PARTIES

(30) Priority: 13.07.2005 US 699416 P
(43) Date of publication of application: 14.05.2008
(73) Proprietor: Medtronic, Inc., Fridley, MN 55432 (US)
(72) Inventor: GURSKIS, Donnell, W., Belmont, California 94002 (US)
(74) Representative: Zimmermann & Partner
(86) International application number: PCT/US2006/027687
(87) International publication number: WO 2007/009117

(56) References cited:
- WO-A-2004/006810
- WO-A-2005/072655
- WO-A2-01/87190
- US-A1- 2003 023 302
- US-A1- 2005 043 760
- US-B1- 6 200 306

## Description

### BACKGROUND

Prosthetic heart valves can replace defective human valves in patients. For example, one piece valves have been suggested that include sewing rings or suture cuffs that are attached to and extend around the outer circumference of a prosthetic valve. In addition, multiple component valves have also been suggested that include a sewing ring that is separate from a valve component. The sewing rings of either type of prosthetic valve can be tedious and time consuming to secure within a target site, i.e., within an annulus of a heart where a natural heart valve has been removed.

For example, to implant a sewing ring within an annulus of a heart, between twelve and twenty sutures may be secured initially to tissue surrounding the annulus. The sewing ring and/or the entire prosthetic valve may then be advanced or "parachuted" down the sutures into the annulus. Knots may then be tied with the sutures to secure the sewing ring within the annulus, whereupon the sutures may be cut and the excess removed. Consequently, this procedure can be very complicated, requiring management and manipulation of many sutures. The complexity of the procedure also provides a greater opportunity for mistakes and requires a patient to be on cardiopulmonary bypass for a lengthy period of time.

Because the annulus of the heart may not match the circular cross-section of the sewing ring and/or prosthetic valve, the prosthetic valve may not fit optimally within the annulus. As a result, natural blood hemodynamics through and around the valve may be impaired, resulting in clotting, possible emboli production, and eventual calcification of the valve structure.

To address this concern, flexible sewing rings have been suggested for use with multiple component valves. The sewing ring may be implanted within the annulus, e.g., using the procedure described above, i.e., parachuted down an arrangement of sutures. The sewing ring may conform at least partially to the anatomy of the annulus. Alternatively, instead of using sutures, it has also been suggested to drive staples through the sewing ring into the surrounding tissue to secure the sewing ring.

When a mechanical or prosthetic valve is then attached to the sewing ring, however, the valve and sewing ring may not mate together effectively, e.g., if the shape of the sewing ring has been distorted to conform to the annulus, which may also impair natural blood hemodynamics, create leaks, and/or otherwise impair performance of the prosthetic valve.

Percutaneous valves have also been suggested that may be delivered using a catheter or other device, e.g., from a percutaneous delivery site. Such valves, however, risk damage to tissue leaflets and/or other components of the valves, e.g., due to the substantial compression and expansion involved during delivery. In addition, such valves may be difficult to attach to a native biological annulus.

US 2005/0043760 describes a biologically implantable prosthesis with engagement elements for engagement between a first and a second element of the prosthesis.

### SUMMARY OF THE INVENTION

The present invention is directed to heart valves that may be implanted within a patient, and, more particularly, to multiple component heart valve assemblies that may be delivered endoluminally into a patient's heart, e.g., from a percutaneous entry site.

In accordance with an embodiment, a system is provided for delivering a multiple component prosthetic valve into a biological annulus within a patient's body that includes an elongate tubular member including a proximal end, a distal end sized for introduction into a body lumen, and a lumen extending between the proximal and distal ends. A first annular prosthesis may be disposed within the tubular member adjacent the distal end in a contracted condition, the annular prosthesis being expandable upon deployment from the tubular member within the biological annulus. A second valve prosthesis may be disposed within the tubular member adjacent the annular prosthesis in a contracted condition, the valve prosthesis being expandable upon deployment from the tubular member within the biological annulus such that the valve prosthesis may be secured to the annular prosthesis.

The system includes a plurality of elongate guide elements including a first end attachable to tissue surrounding a biological annulus and having sufficient length to extend from the biological annulus to a percutaneous entry site. The guide elements may be sized to be received through at least one of the tubular member, the annular prosthesis, and the valve prosthesis. For example, the valve prosthesis may include a plurality of passages for receiving respective guide elements therethrough such that the valve prosthesis may be advanced over the guide elements. In addition or alternatively, the annular prosthesis may include a plurality of passages for receiving respective guide elements therethrough such that the annular prosthesis may be advanced over the guide elements. Alternatively, the guide elements may be secured to the annular prosthesis. Optionally, each of the guide elements may include one or more connectors spaced apart from the first end for securing at least one of the annular prosthesis and the valve prosthesis relative to tissue to which the first end is attached.

In one embodiment, the system may include one or more pusher members disposed within the tubular member, the pusher member(s) adjacent at least one of the annular and valve prostheses, and being movable relative to the tubular member for deploying at least one of the annular and valve prostheses.

Other aspects and features of the present invention will become apparent from consideration of the following description taken in conjunction with the accompanying drawings.

### BRIEF DESCRIPTION OF THE DRAWINGS

Figures 1 to 6 illustrate exemplary embodiments of the invention, in which:
FIG. 1 is a perspective view of a two piece heart valve assembly including a gasket member and a valve member that has been partially folded.
FIGS. 1A and 1B are details showing a method for folding the valve member of FIG. 1 into a compressed delivery condition.
FIG. 2 is a cross-sectional view of a patient, showing a method for delivering the heart valve assembly of FIG. 1.
FIGS 3A-3C are perspective views of another two piece heart valve assembly including a gasket member and a valve member, showing the valve member being directed towards the gasket member over guide elements and secured to the gasket member using connectors on the guide elements and receiving tubes of the valve member over posts of the gasket member.
FIG. 4 is a detail showing an alternative structure for securing the valve member to the gasket member, including a plurality of ratcheting elements on a guide member.
FIGS. 5-5C are cross-sectional views of a patient, showing a method for implanting a heart valve assembly into a biological annulus.
FIG. 6 is a detail showing another alternative structure for securing a valve member to a gasket member, including cooperating connectors on the valve member and gasket member.
FIGS. 7A and 7B are perspective views of a heart valve member in compressed and expanded conditions, respectively.
FIGS. 8A and 8B are details of posts that may be provided on the heart valve member of FIGS. 7A and 7B.
FIGS. 9A and 9B are details of alternative posts that may be provided on a heart valve member, such as that shown in FIGS. 7A and 7B.
FIGS. 10A and 10B are front and top views, respectively, of a commissure of a heart valve member with leaflets attached thereto.
FIGS. 11A and 11B are front and top views, respectively, of a commissure of a heart valve member with leaflets attached thereto.

### DETAILED DESCRIPTION OF THE PREFERRED EMBODIMENTS

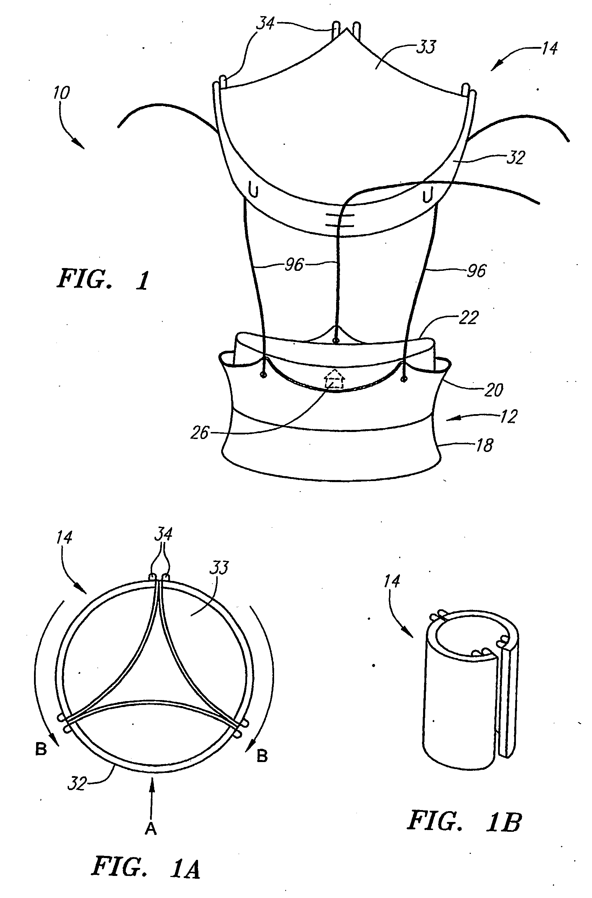

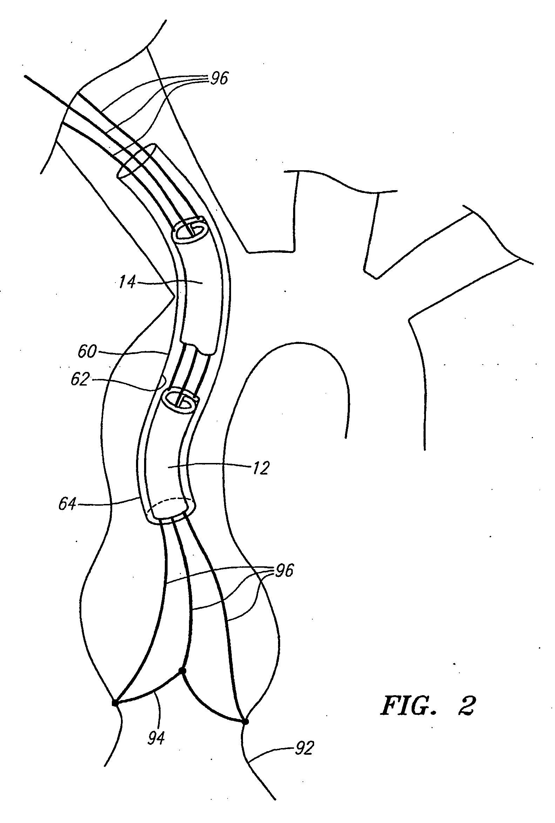

Turning to the drawings, FIG. 1 shows an exemplary embodiment of a heart valve assembly 10 that generally includes a base member or "gasket member" 12 and a valve member or "crown" 14. In addition, as shown in FIG. 2, filaments, sutures, or other elongate guide elements 96 may also be provided, e.g., in a system or kit, to guide the gasket member 12 and/or valve member 14 into an implantation site (not shown) and/or to secure the valve member 14 to the gasket member 12, as described further below.

As shown in FIG. 1, the valve member 14 may include an annular shaped body or frame 32 and one or more valve elements 33. In an exemplary embodiment, the valve member 14 is a bioprosthetic valve, i.e., an annular frame 32 carrying a plurality of tissue leaflets 33 extending from the frame 32, e.g., attached to commissures 34. The frame 32 may have a noncircular, e.g., multiple lobular shape, such as a tri-lobular shape, including three lobes separated by cusps or scallops. The frame 32 and/or other components of the valve member 14 may include a fabric covering (not shown), e.g., to enhance sealing and/or facilitate tissue ingrowth, which may be sutured or otherwise secured around, over, or otherwise to the component(s).

The valve member 14 may include a plurality of struts (not shown) that may be attached to the frame 32 and/or otherwise carry the leaflets or other valve elements 33. For example, the struts may include a laminate structure, including two or more sheets of flexible material, similar to the struts disclosed in U.S. Patent No. 6,371,983 ("the '983 Patent"). The leaflets may be formed from tissue, such as bovine pericardium, as described in the '983 Patent. Exemplary leaflets and methods for assembling them into crowns are described in co-pending application Serial No. 11/144,254, filed June 3, 2005.

Alternatively, the valve member 14 may be a connecting device to which a valve (not shown) may be connected or that may otherwise receive a valve component, such as the connection adapter elements shown in co-pending application Serial No. 10/646,639, filed August 22, 2003. In further alternatives, the valve member 14 may include a mechanical valve or other valve (not shown), such as those disclosed in application Serial Nos. 10/765,725, filed January 26, 2004, 11/069,457, filed February 28, 2005, and 60/669,704, filed April 8, 2005.

Turning to FIGS. 1A and 1B, the valve member 14 may be contractible into a contracted condition to facilitate introduction into a catheter or other delivery device (not shown), e.g., to allow delivery endoluminally through a patient's vasculature, as explained further below. For example, as shown in FIG. 1A, the valve member 14 may have an enlarged or relaxed condition, which may correspond to the desired configuration for the valve member 14 once it is implanted and in use within a biological annulus, e.g., above a native aortic, mitral, pulmonary valve site within a patient's heart. As shown in FIG. 1, the frame 32 may be folded out of plane (e.g., as represented by arrow A in FIG. 1A), thereby generally defining two generally semi-circular portions adjacent one another. The opposite ends of the frame 32 may then be rolled towards one another (e.g., as represented by arrows B in FIG. 1A), until the valve member 14 is folded or rolled into the contracted condition shown in FIG. 1B. In the contracted condition, the valve member 14 may be loaded into a catheter or other delivery device (not shown), as explained further below.

Returning to FIG. 1, the gasket member 12 generally includes an annular ring 18, and a sewing cuff 20 extending radially outwardly from the annular ring 18. Optionally, the gasket member 12 may also include a collar or stand-off 22, such as those disclosed in application Serial No. 60/685,265, filed May 27, 2005. A fabric covering may be provided on one or more components of the gasket member 12, e.g., to enhance sealing and/or facilitate tissue ingrowth. In one embodiment, the annular ring 18 may have a generally circular shape, although alternatively, the annular ring 18 may have a multi-lobular shape about the circumference, e.g., including three lobes separated by scallops or cusps (not shown).

The annular ring 18 may be formed from an elastic or superelastic material, such as Nitinol, Elgiloy, stainless steel, and the like. For example, the annular ring 18 may be cut from a flat sheet of base material having a desired thickness for the annular ring 18, for example, by laser cutting, mechanical cutting, and the like. Thus, the annular ring 18 may be initially formed as a long band of material, having a width corresponding to the desired width of the annular ring 18. The band may be wrapped around a mandrel or otherwise restrained in a generally cylindrical shape with the ends adjacent to one another, and the band may be heat treated or otherwise processed to program the generally cylindrical shape to create the annular ring 218. The generally cylindrical shape may include the ends overlapping one another, spaced apart from one another to provide an open "C" shape, or attached to one another.

When the annular ring 18 is at least partially covered with fabric, the fabric may be wrapped around the annular ring 18, while accommodating expansion and contraction of the annular ring 18. For example, at least near the ends, the fabric may not be secured to the annular ring 18, allowing the ends to slide circumferentially relative to the fabric. Optionally, sutures and the like (not shown) may be used to secure the fabric to the annular ring 18 at locations removed from the ends, e.g., at one or more intermediate locations about the circumference of the annular ring 18. Alternatively, the entire annular ring 18 may be free to slide within the fabric wrapped around the annular ring 18.

The sewing cuff 20 may be attached to or otherwise extend around the annular ring 18. The sewing cuff 20 may simply be a layer of fabric or other material covering at least a portion of the annular ring 18. Alternatively, the sewing cuff 20 may include flexible core material (not shown) that may be attached to or otherwise extend around the annular ring 18, e.g., from an upper edge of the annular ring 18. If the gasket member 12 includes the collar 20, the collar 22 may be attached to or otherwise extend upwardly from the annular ring 18 and/or the sewing cuff 20. Optionally, the collar 22 and sewing cuff 20 may include a core that is formed as a unitary piece or attached together. The material of the core may be substantially flexible, e.g., manufactured in a desired annular shape, yet easily deformed, e.g., deflected, stretched, and/or compressed. Exemplary materials for the core include silicone or other elastomeric materials, foam, fabric, felt, polymers, and the like. The materials may be molded or otherwise formed into the core, e.g., using known molding, extrusion, cutting, or other manufacturing procedures.

Optionally, the gasket member 12 may include one or more attachment zones 26 (one shown in phantom). The attachment zone(s) 26 may include a connector for securing the valve member 14 relative to the gasket member 12 and/or may define an area of where the valve member 14 contacts the gasket member 12 when the valve member 14 is secured relative to the gasket member 12, e.g., to provide a desired seal.

As shown in FIGS. 1 and 2, the gasket member 12 may be contractible into a contracted condition to facilitate delivery. In one embodiment, the gasket member 12 may be biased to expand to a predetermined diameter, e.g., to an enlarged condition corresponding to the biological annulus within which the gasket member 12 is to be implanted. The gasket member 12 may be compressed, e.g., by flattening the annular ring 18, and then rolling the ends, similar to the crown 14, as described above. The gasket member 12 may then be loaded into a catheter 60 or other delivery device (not shown), e.g., adjacent the crown 14.

Alternatively, the gasket member 12 may be compressed into a contracted condition resembling a clover, e.g., by directing multiple portions of the annular ring 18 radially inwardly relative to adjacent portion, thereby defining a plurality of petals. This configuration may be sufficiently small to allow the gasket member 12 to be loaded into a delivery device. Optionally, the petals may be folded together to further compress the gasket member 12. Exemplary contracted configurations are shown in US application Serial No. 60/746,038, filed April 29, 2006. In another alternative, the frame 32 of the valve member 14 may be compressed in a similar manner, taking care not to damage the leaflets 33.

In addition, the frame 32 and/or other component of the valve member 14 may include one or more connectors for securing the valve member 14 relative to the gasket member 12. Similarly, the gasket member 12 may include one or more mating connectors and/or receivers for cooperating with the connectors on the valve member 14. Alternatively, the valve member 14 and/or gasket member 12 may include one or more receivers for receiving a suture or other filament therethrough, e.g., to guide and/or secure the valve member 14 and/or gasket member 12, as described further below. Optionally, the valve member 14 and/or gasket member 12 may include one or more radiopaque markers or other guiding elements, e.g., that may be visualized using fluoroscopy or other external imaging to facilitate positioning and/or implantation of the valve member 14 and/or gasket member 12.

Turning to FIG. 2, an exemplary method is shown for delivering a two-component heart valve assembly 10, such as that described above. As shown, the heart valve assembly 10 may include a gasket member 12 and a valve member 14 loaded into a catheter 60, thereby providing a system that may be used to deliver and/or implant the heart valve assembly 10 into a biological annulus 92,e.g., to replace a native aortic valve 94. In an exemplary embodiment, the gasket member 12 and valve member 14 are disposed adjacent one another within a lumen 62 of the catheter 60 adjacent to a distal end 64, which may be tapered or otherwise sized and/or shaped to facilitate introduction into a patient's vasculature or other body lumens.

As shown, the gasket member 12 is disposed immediately adjacent the distal end 64 and the valve member 14 is disposed proximal to the gasket member 12 such that the gasket member 12 and valve member 14 may be delivered successively from the catheter 60. Optionally, the catheter 60 may include one or more pusher members (not shown) adjacent to the gasket member 12 and/or valve member 14, which may be used to deploy the gasket member 12 and/or valve member 14. For example, the pusher member(s) may include one or more tubular bodies or other structures (not shown) that restrain the gasket member 12 and/or valve member 14 from axial movement when the catheter 60 is retracted, thereby exposing the gasket member 12 and crown 14 beyond the distal end 64.

For example, a first pusher member may be provided that includes a distal end disposed adjacent the gasket member 12, and a second pusher member may be provided that includes a distal end disposed adjacent the valve member 14. The first pusher member may include a lumen, slot, or other feature for accommodating the valve member 14 and/or second pusher member. Thus, if the catheter 60 is withdrawn proximally while maintaining the first pusher member substantially stationary, the gasket member 12 may be deployed from the distal end 64 of the catheter 60, while the second pusher member and valve member 14 move with the catheter 60. Thereafter, the catheter 60 may be withdrawn further proximally while maintaining the second pusher member substantially stationary, the valve member 14 may be deployed from the distal end 64 of the catheter 60. Alternatively, a single pusher member may be provided that may carry the gasket member 12 and valve member 14, thereby allowing the gasket member 12 and valve member 14 to be deployed successively from the catheter 60.

After manufacturing the components of the heart valve assembly 10 and catheter 60, the components may be loaded into the catheter 60, e.g., during manufacturing or any time before delivery into a patient. In an exemplary embodiment, the gasket member 12 and valve member 14 may be contracted or folded separately from each other and loaded successively into the catheter 60, e.g., from the distal end 64. If the catheter 60 includes a pusher member for carrying the components, the gasket member 12 and valve member 14 may be loaded onto the pusher member, which may then be inserted into the catheter 60. One of the advantages of separating the gasket member 12 and valve member 14 and loading them separately is that they may be folded or otherwise compressed to a smaller diameter or size than if folded after the gasket member 12 and valve member 14 are attached to each other. Furthermore, folding the gasket member 12 and valve member 14 separately may reduce stress on the gasket member 12 and valve member 14 while contracted, which may improve durability of the heart valve assembly 10.

Guide elements (not shown) are loaded into the catheter 60 during manufacturing and/or assembly. For example, if the gasket member 12 and/or valve member 14 include tubular members or other features (not shown) for receiving the guide elements therethrough, the guide elements may be directed through the features before loading the gasket member 12 and/or valve member 14 into the catheter 60. The guide elements are directed through the catheter 60, e.g., to the proximal end (not shown), e.g., such that the guide elements extend from the proximal end, through the valve member 14 and/or gasket member 12 (e.g., through lumen 62) and to the distal end 64 of the catheter 60. The guide elements extend a predetermined distance out of the distal end 64 of the catheter 60, e.g., to provide sufficient length that may introduced into a patient before the catheter 60.

Turning to FIG. 2, during use, the guide elements 96 are delivered and attached to tissue surrounding the biological annulus 92.

In an exemplary embodiment, the sutures 96 may be delivered from a percutaneous entry site, e.g., a puncture in the femoral, carotid, radial, or other artery, into the aortic root. From within the aortic root, the sutures may be driven through or otherwise secured to the tissue surrounding the biological annulus 92. If desired, knots may be directed down the sutures 96 to the location where they are secured to the tissue surrounding the biological annulus 92. Alternatively, the sutures 96 may include two ends that extend from the biological annulus 92. Thus, the sutures 96 may extend from the biological annulus 92, through any intervening vasculature to the percutaneous entry site, and out of the patient's body.

Once the sutures 96 are in place, the catheter 60 may be introduced through the percutaneous entry site, and advanced through the patient's vasculature over the sutures 96 into the aortic root. For example, free ends of the sutures 96 may be backloaded into the distal end 64 of the catheter 60, through the gasket member 12 and/or valve member 14, and the catheter 60 to the proximal end. Alternatively, as described above, the sutures 96 may be preloaded through the catheter 60. Optionally, the catheter 60 may be inserted through an introducer sheath or other device (not shown) at the entry site, using known procedures.

With the distal end 64 of the catheter 60 positioned adjacent or within the biological annulus 92, the gasket member 12 may be deployed from the distal end 64, e.g., by retracting the catheter 60 partially and using a pusher member or otherwise preventing proximal movement of the gasket member 12. Upon being exposed within the biological annulus 92, the gasket member 12 may resiliently return to its expanded or relaxed condition. Alternatively, the gasket member 12 may be expanded or "unfurled" as it is advanced distally along the sutures 96. In a further alternative, a tool (not shown) may be advanced over the sutures 96 or over the catheter 60, which may be used to expand the gasket member 12.

The gasket member 12 may then be seated within the biological annulus 92. For example, the pusher member (not shown) may be advanced to direct the gasket member 12 over the sutures 96, e.g., into the site of the native valve leaflets 94. If the native valve leaflets 94 remain within the biological annulus 92 during delivery, the gasket member 12 may deflect the leaflets 94 outwardly to open the biological annulus 92. Optionally, the gasket member 12 may at least partially dilate the biological annulus 92, similar to the methods in the applications identified above, e.g., because of the resilient bias of the annular ring 18 to expand radially outwardly.

For example, with the annular ring 18 contracted into a relatively small diameter (if the annular ring 18 is radially compressible), the gasket member 12 may be advanced into the biological annulus 92 using a delivery tool (not shown). The gasket member 12 may be advanced until the annular ring 18 extends at least partially into the biological annulus 92. In one embodiment, the annular ring 18 and/or other component of the gasket member 12 may extend entirely through the biological annulus, with the lower edge of the annular ring 18 remaining free within the sub-annular space below the biological annulus 92. Optionally, the gasket member 12 may include a flexible skirt (not shown) that may extend below through the biological annulus 92 when the gasket member 12 is secured. The skirt may be biased to extend outwardly, e.g., to provide a smooth transition and/or enhance a seal between the heart vale assembly 10 and the biological annulus 92.

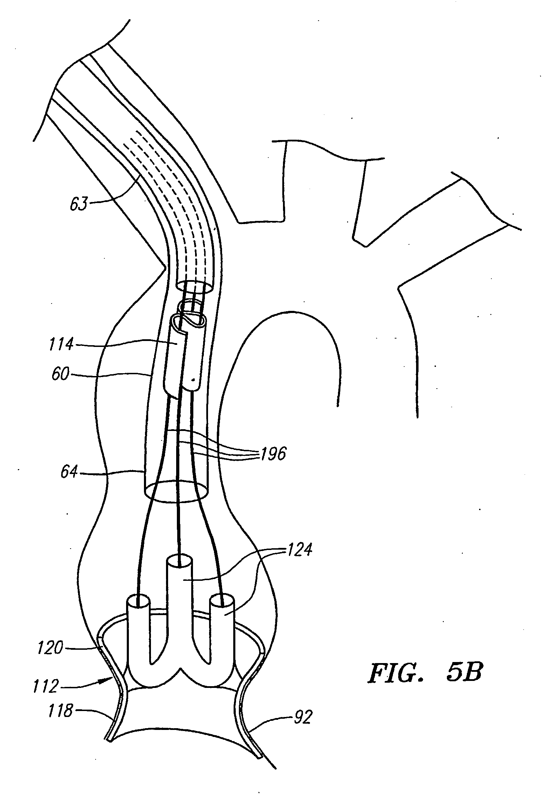

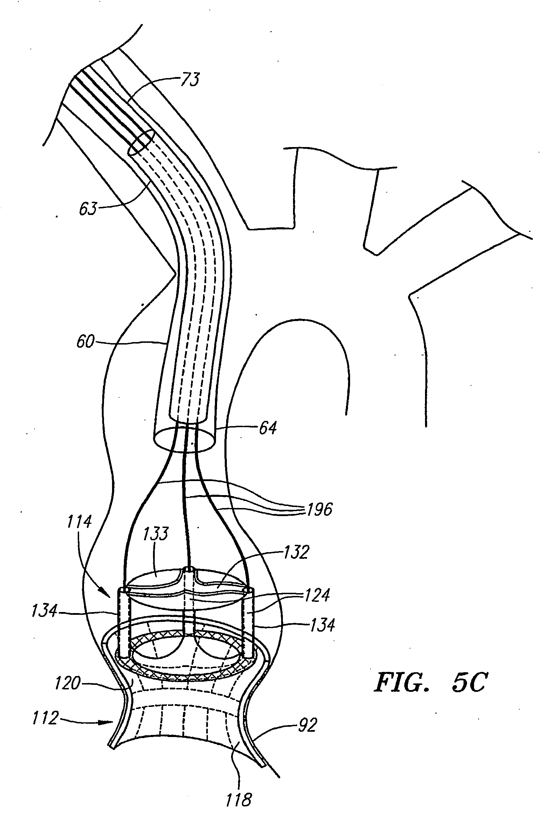

Similar to the embodiment shown in FIG. 5B, the sewing cuff 20 may contact the tissue within the supra-annular space above the biological annulus 92, although the sewing cuff 20 may not provide any structural support of the annular ring 18.

If the annular ring 18 is expandable or otherwise compressed, the annular ring 18 may then be expanded within the biological annulus, e.g., to dilate the biological annulus or otherwise direct the surrounding tissue outwardly against the underlying tissue structures. Alternatively, a dilation tool (not shown) may be advanced into the gasket member 12 and expanded to forcibly (e.g., plastically) expand the annular ring 18 within the biological annulus 92. As the gasket member 12 is delivered, the sewing cuff 20 may be released to allow the sewing cuff 20 to contact the surrounding tissue, e.g., within the aortic root above the biological annulus 92. Optionally, the sewing cuff 20 may adopt the shape of the surrounding tissue, e.g., lying flatter within the coronary sinus regions, while becoming more vertical adjacent the commissures.

The gasket member 12 may be secured within the biological annulus 92 simply by the frictional engagement between the annular ring 18 and the surrounding tissue. Alternatively, the annular ring 18 and/or sewing cuff 20 may include one or engagement elements (not shown) that puncture or otherwise engage the surrounding tissue to enhance securing the gasket member 12. In a further alternative, a plurality of fasteners, e.g., clips, staples, sutures, and the like, may be directed through the sewing cuff 20 into the tissue surrounding the biological annulus 92 to secure the gasket member 12 relative to the biological annulus 92.

With continued reference to FIG. 2, the valve member 14 may then be deployed from the catheter 60 (or from a separate delivery device after delivering fasteners through the gasket member 12) and advanced into the biological annulus 92. For example, the valve member 14 may be advanced over the sutures 96 until connectors on the gasket member 12 and/or valve member 14 engage to secure the valve member 14 to the gasket member 12. For example, the valve member 14 and/or gasket member 12 may include one or more cooperating clips, detents, and the like that may self-engage when the valve member 14 is docked against the sewing cuff 20 or otherwise into the gasket member 12, similar to the embodiments described in the applications identified above.

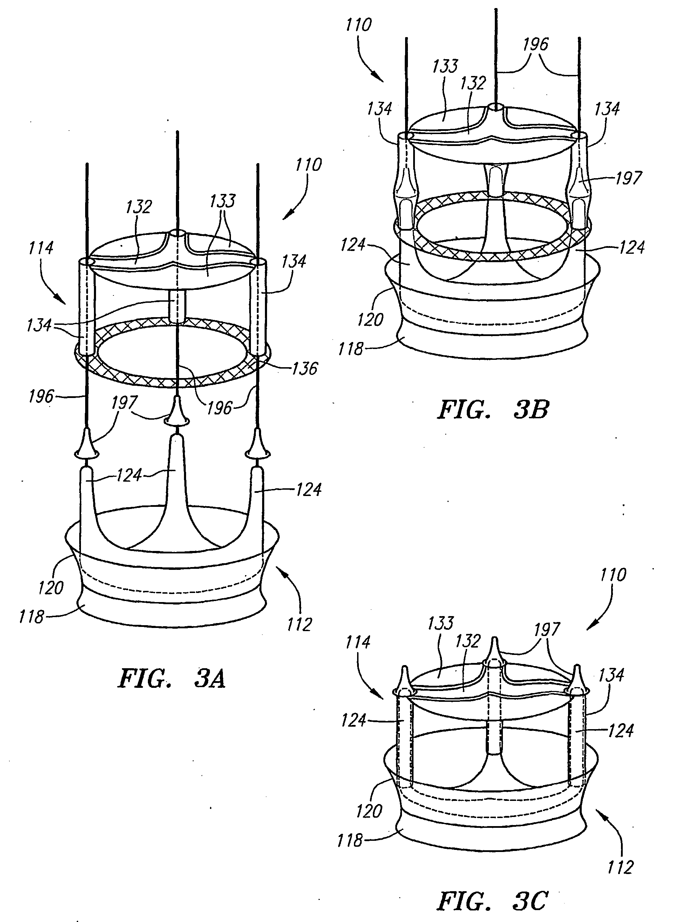

Alternatively, the valve member 14 may be secured to the collar 22, e.g., using one or more connectors on the valve member 14 and/or collar 22, e.g., a drawstring (not shown). In a further alternative, the sutures 96 may be used to secure the valve member 14 to the gasket member 12, similar to the embodiment shown in FIGS. 3A and 3B and described further elsewhere herein.

Turning to FIGS. 3A-3C, another embodiment of a heart valve assembly 110 is shown that includes a gasket member 112 and a valve member or crown 114, which may be constructed similar to the other embodiments described herein. In this embodiment, the gasket member 112 includes an annular ring 118, and a sewing cuff 120, which may be similar to the previous embodiments. In addition, the gasket member 112 includes a plurality of posts 124 extending upwardly, e.g., from the annular ring 118.

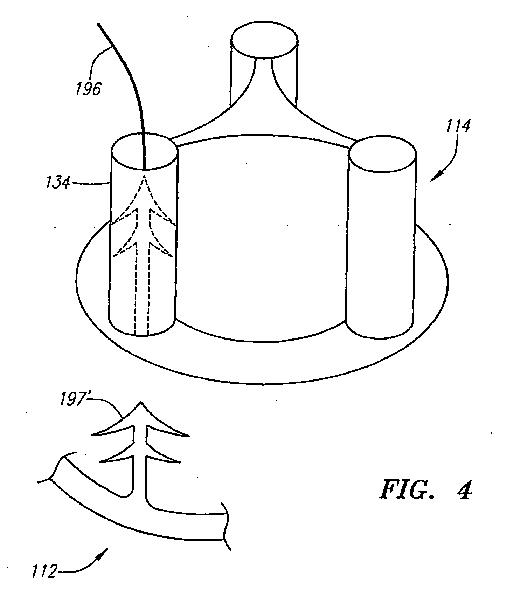

The posts 124 may include passages, tubular elements, or other receivers (not shown) for receiving sutures or other filaments 196 therethrough. Alternatively, the sutures 196 may be attached to ends or other portions of the posts 124. In this alternative, the sutures 196 may include detents or other connectors 197 disposed a predetermined distance from the ends of the posts 124. For example, the connectors 197 may be formed from plastic or metal components fixed to the sutures 196 and including ramped or tapered proximal surfaces and blunt distal surfaces or edges. In the embodiment shown in FIGS. 3A and 3B, the connectors 197 are conical features. Alternatively, as shown in FIG. 4, the connectors 197' may be ratcheting features, e.g., including harpoon tips and/or one or more, e.g., a plurality of one-way ratchets.

Returning to FIGS. 3A-3C, the valve member 114 includes a sleeve 132, e.g., of bovine pericardium or other tissue, synthetic material, fabric, and the like, which may be formed to provide a plurality of leaflets 133. The sleeve 132 may also include a plurality of tubes or other receivers 134, which may be formed the same material as the leaflets 133, e.g., bovine pericardium, and/or other material, e.g., fabric. For example, the tubes 134 may be formed by bonding, stitching, and/or otherwise attaching portions of the sleeve 132 to create the tubes 134 between adjacent leaflets 133. Alternatively, the tubes 134 may be formed from separate material attached to the sleeve 132, e.g., cloth, silicone, and/or other material that may be flexible, for example, so that the tubes 134 may be folded and/or expanded easily. The tubes 134 may be sized to be received over the posts 124 of the gasket member 112, as explained further below.

Optionally, the valve member 114 may include a cloth ring 136 at the base of the tubes 134, e.g., for providing a tissue ingrowth surface. The cloth ring 136 may be formed from cloth and, optionally, may include a silicone or other core (not shown), which may be attached to the sleeve 132, e.g., by stitching, bonding, and the like. The cloth ring 136 may provide an additional interface with the gasket member 112, e.g., to enhance sealing when the valve member 114 is secured relative to the gasket member 112.

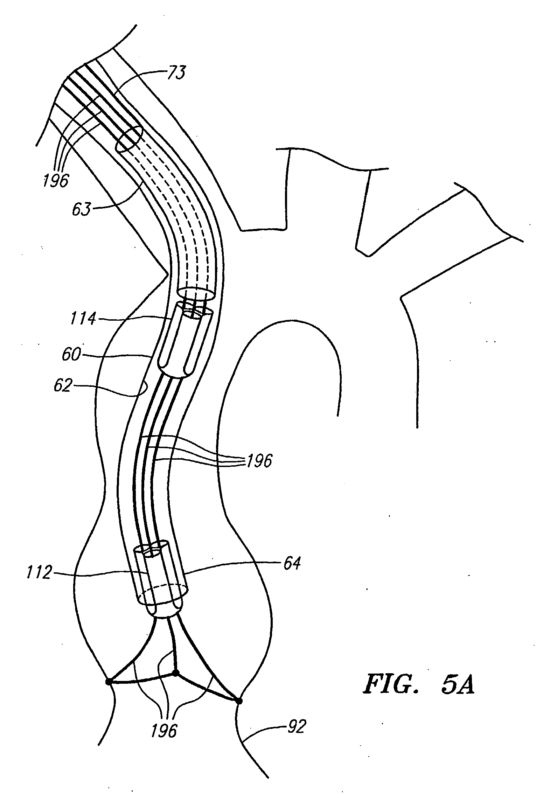

Turning to FIGS. 5A-5C, the gasket member 112 and valve member 114 may be loaded into a catheter 60, similar to the previous embodiments. Optionally, the catheter 60 may include a pusher member 63 adjacent to the gasket member 112 and/or valve member 114, which may be used to deploy the gasket member 112 and/or valve member 114, similar to embodiments described elsewhere herein. As shown in FIG. 5A, a pusher wire 73 may be attached to the pusher member 63, which may extend proximally through the catheter 60. The pusher wire 73 may be coupled to an actuator, e.g., a slider, button, dial, or other feature (not shown), on a handle (also not shown) on the proximal end of the catheter 60. Thus, the actuator and pusher wire 73 may be used to hold or push the pusher member 63 relative to the catheter 60, e.g., along the catheter lumen 62.

As shown in FIG. 5A, with the components of heart valve assembly loaded in the catheter, the catheter 60 may be advanced over sutures 196 previously attached to tissue surrounding the biological annulus 92, e.g., as described above. Once the distal end 64 of the catheter 60 is disposed adjacent the biological annulus 92, e.g., within the aortic root for aortic valve replacement, the gasket member 112 may be deployed from the catheter 60 and advanced over the sutures 196 into the biological annulus 92, similar to the methods described above. If the sutures 196 include the connectors 197, such as those shown in FIGS. 3A-3C (and not shown in FIGS. 5A-5C), the gasket member 112 may be advanced such that the connectors 197 pass through passages or receivers in the gasket member 112. For example, the proximal end of the passages in the gasket member 112 may include a narrow region, one or more tabs, and the like (not shown) that may allow the connectors 197 to pass through as the gasket member 112 is advanced, but prevent the gasket member 112 from being withdrawn back over the connectors 197. Alternatively, the sutures 196 and connectors 197 may simply pass through a portion of the fabric covering on the gasket member 112, e.g., covering the sewing cuff 120.

With the gasket member 112 delivered into the biological annulus 92, the valve member 114 may be deployed from the catheter 60, e.g., similar to the previous methods, and advanced over the sutures 196 until the valve member 114 is docked to the gasket member 112. With additional reference to FIG. 3C, as the valve member 114 is advanced over the connectors 197, the connectors 197 may pass through the tubes 134 of the valve member 14 until the connectors 197 exit from the proximal or upper ends of the tubes 134. This advancement may be facilitated by the ramped proximal surfaces of the connectors 197. The blunt lower edges of the connectors 197 may then abut the proximal ends of the tubes 134 or otherwise prevent the valve member 114 from being removed back over the connectors 197. Thus, the valve member 114 may be substantially secured relative to the gasket member 112, e.g., against the gasket member 112, as shown in FIG. 3C.

The excess suture material 196 above the connectors 197 may then be removed, leaving the valve member 114, gasket member 112, and connectors 197, and sutures secured to the tissue within or adjacent the biological annulus 92. For example, in one embodiment, the sutures 196 may include weakened or break-away areas, e.g., immediately above the connectors 197 that may separate when a threshold tensile force is applied to the sutures 196. Alternatively, a cutter or other tool (not shown) may be advanced into the aortic root (or other region adjacent the biological annulus 92) and manipulated to cut or otherwise sever the sutures 196 above the connectors 197.

One advantage with this embodiment is that the crown 114 may simply be a sleeve 132 formed from tissue, and may not include a frame, unlike the previous embodiments. This may facilitate rolling or otherwise contracting the crown 114 into a delivery condition. In addition, the embodiment may eliminate any risk of metal or other frame components damaging tissue leaflets during contraction and/or expansion. Thus, the posts 124 on the gasket member 112 provide a frame-like support structure that supports the sleeve 132 and allows the leaflets of the sleeve 132 to open and close during beating of the heart.

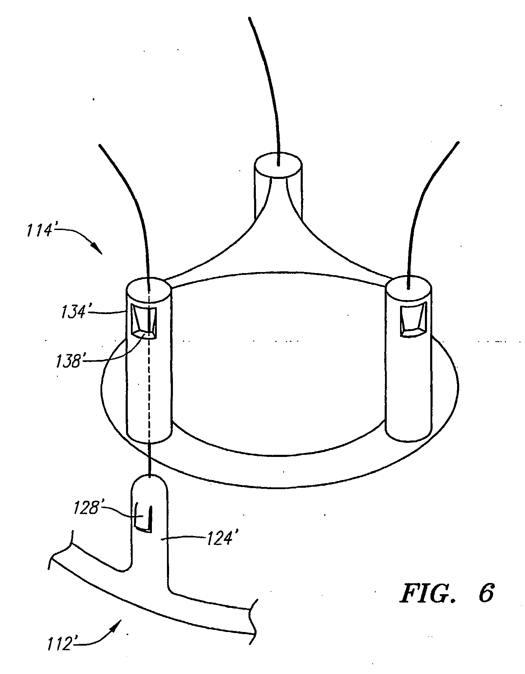

In other embodiments, such as that shown in FIG. 6, posts 124' on a gasket member 112' may include detents, tabs, or other connectors 128' that may accommodate receiving a portion of a valve member 114,' e.g., tubes 134' over the posts 124.' The tubes 134' may include receptacles 138' that interlock or otherwise engage with the connectors 128' to secure the valve member 114' to the gasket member 112.' For example, as shown, the tabs 128' may present a tapered edge that allows the tubes 134' to pass freely over the posts 124,' yet is biased to resiliently return outwardly to prevent removal or other movement of the tubes 134' off of the posts 124.' The receptacles 138' receive the outwardly returned tabs 128'.

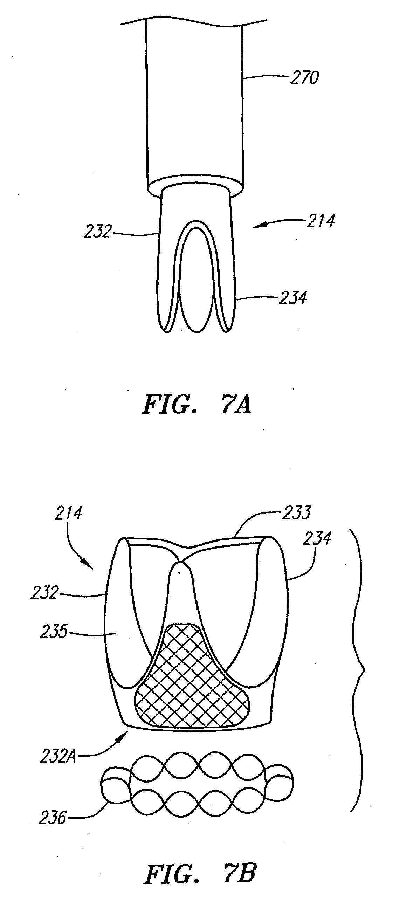

Turning to FIGS. 7A and 7B, a valve member or crown 214 is shown that includes an expandable frame 232 that may be compressed inwardly into a delivery condition (shown in FIG. 7A) and expandable to a deployed condition (shown in FIG. 7B). As shown, the valve member 214 may include an annular shaped body or frame 232, one or more valve elements 233, and a base 232a. The valve member 214 is a bioprosthetic valve member, i.e., an annular frame 232 carrying a plurality of tissue leaflets 233 extending from the frame 232, e.g., attached to commissures 234. The frame 232 may have a noncircular, e.g., multiple lobular shape, such as a tri-lobular shape, including three lobes separated by cusps or scallops. Optionally, the frame 232 may include one or more connectors (not shown) for mating with a cooperating connector on a gasket member (also not shown), similar to other embodiments described elsewhere herein.

The frame 232 and/or other components of the valve member 214 may include a fabric covering 235, e.g., to enhance sealing and/or facilitate tissue ingrowth, which may be sutured or otherwise secured around, over, or otherwise to the component(s). The fabric covering 235 comprises a polyester material, e.g., Dacron. The frame 232 may be formed from one or more rod elements, e.g., that have portions removed to provide a desired flexibility for the frame 232. An example uses a solid Nitinol bar that is machined using methods known in the art to create the frame 232, e.g., conventional machining, electrical discharge machining (EDM), laser, and water jet machining. Alternatively, the frame 232 may include a stent member 236 attached to the base 232a of the valve member 214 for increasing expandability of the valve member 214 within a gasket member. The stent member 236 may comprise materials similar to those described above for making annular rings, e.g., Nitinol or other elastic or superelastic material.

The valve member 214 may include a plurality of struts (not shown) that may be attached to the frame 232 and/or otherwise carry leaflets or other valve elements 233. For example, the struts may include a laminate structure, similar to previous embodiments. The leaflets 233 may be formed from tissue, such as bovine pericardium, also similar to previous embodiments. Alternatively, the valve member 214 may be a connecting device to which a valve 233 may be connected or that may otherwise receive a valve component, as described elsewhere herein. In further alternatives, the valve member 214 may include a mechanical valve or other valve (not shown), also as described elsewhere herein.

The valve member 214 may be compressible into the delivery condition using a valve holder tool 270, shown in FIG. 7A, e.g., to facilitate introduction into a delivery catheter (not shown) and/or deployment from the delivery catheter. As shown in FIG. 7A, the frame 232 may be folded inwardly using the tool 270, bringing each of the commissures 234 inwardly towards one another. For example, the tool 270 may include a central core member (not shown) including a plurality of lobes, and a plurality of movable arms disposed between adjacent lobes (also not shown). The frame 232 may be disposed between the core member and the arms, and the arms may be directed inwardly to fold the frame 232, e.g., similar to tools disclosed in US application Serial No. 60/746,038.

With the frame 232 folded or otherwise compressed, the tool 270 and valve member 214 may be loaded into a delivery catheter (not shown). The tool 270 may have an elongate flexible or semi-rigid body (not shown), which may extend through the delivery catheter, yet allow the delivery catheter to be advanced through a patient's vasculature. As shown in FIG. 7B, when it is desired to deploy the valve member 214, e.g., within a biological annulus, the valve member 214 and tool head may be advanced from the delivery catheter, and the frame 232 may be expanded, e.g., by releasing the arms or other actuator on the tool. Upon being released, the frame 232 may resiliently expand, e.g., the commissures 234 may automatically separate from one another to open the leaflets 233. The valve member 214 may then be secured relative to a gasket member (not shown), similar to the other embodiments described elsewhere herein.

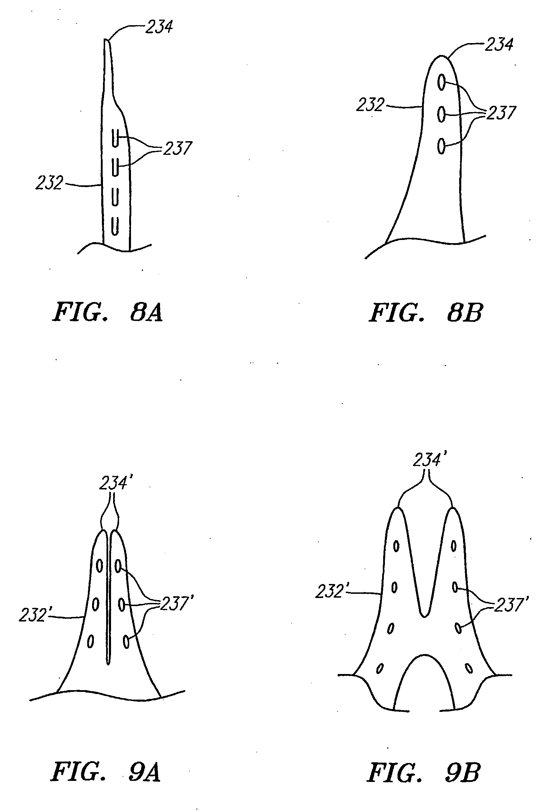

Turning to FIG. 8A and 8B, shown are details of a commissure 234 that may be provided on the frame 232 of the valve member 214 shown in FIGS. 7A and 7B. The frame 232 (or struts, not shown, attached to the frame 232) may include a plurality of holes or other commissure attachment points 237. Sutures or other connectors (not shown) may be directed through the holes 237 to attach a leaflet (also not shown) to the commissure 234, similar to the previous embodiments. Optionally, the thickness of the commissure 234 may be varied to provide a desired stiffness. For example, the tip of the commissure 234 may be thinner than base portions of the frame 232, e.g., to allow greater flexibility of the tip of the commissure 234 for upper edges of leaflets (not shown) attached to the frame 232. The plurality of commissure attachment points 237 provide alternative locations for attaching tissue leaflets (not shown) to the frame 232.

FIGS. 9A and 9B show an example of a frame 232.' The frame 232' includes pairs of commissures 234' (one shown) spaced apart around the circumference of the frame 232,' each commissure including a plurality of holes or other commissure attachment points 237.' Each pair of commissures 234' may support edges of individual, adjacent leaflets (not shown) such that each leaflet is independently supported. The commissures 234' may be directed inwardly towards one another (as shown in FIG. 9A) as the frame 232' is contracted to the delivery condition and may resiliently move away from one another (as shown in FIG. 9B) when the frame 232' is released, e.g., during deployment and implantation of the heart valve assembly, similar to the previous embodiments.

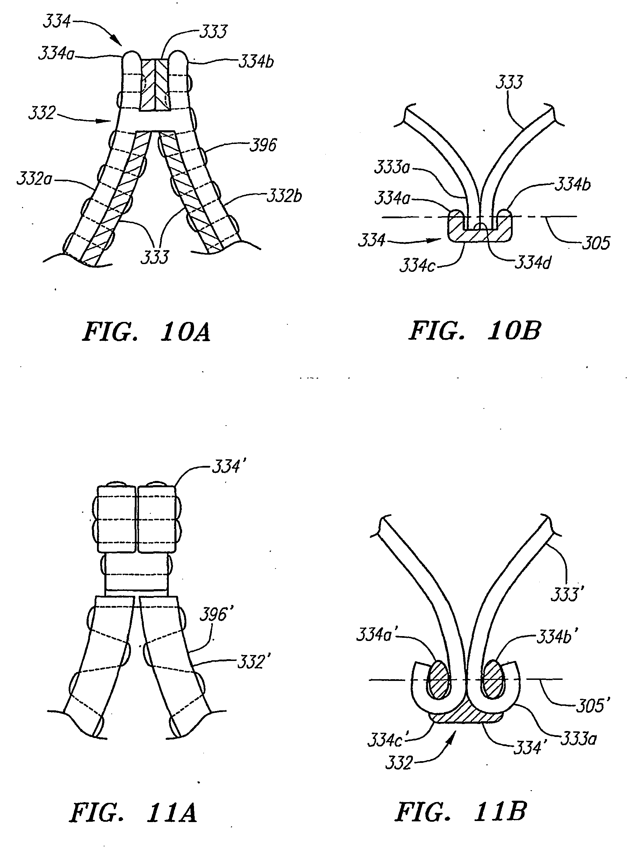

FIGS. 10A-11B show alternative constructions and methods for attaching leaflets to an expandable frame, e.g., to provide a valve member (not shown), such as those described elsewhere herein. FIGS. 10A and 10B show a portion of a frame 332 that includes a commissure 334 to which a leaflet 333 is attached. As best seen in FIG. 10B, the edges of the leaflets 333 may be received within a channel 334d formed in the commissure 334, e.g., defined by two sides 334a, 334b and a backside 334c. The edges 333a of the leaflets 333 may abut into the channel 334d. One or more sutures 396 (as seen in FIG. 10A) may then be applied along a suture line 305, e.g., through the leaflets 333 and the commissure 334, to secure the leaflets 333 to the frame 332. The commissure 334 may include one or more holes, slots, and the like (not shown), similar to those shown in FIGS. 8A-9B, for receiving the sutures 396 therethrough.

Turning to FIGS. 11A and 11B, another construction and method for attaching leaflets to a frame is shown. As shown in FIG. 11A, a frame 332' may include a plurality of commissures 334' spaced apart around a circumference of the frame 332.' Each commissure 334' may include two sides 334a', 334b' and a backside 334c.' The two sides 334a', 334b' may extend upwardly beyond the backside 334c,' thereby defining posts around which edges 333a' of valve leaflets 333' may be wrapped. Sutures 396' may then be applied along the suture line 305' to secure the leaflets 333' to the frame 332,' similar to the previous embodiments.

While the invention is susceptible to various modifications, and alternative forms, specific examples thereof have been shown in the drawings and are herein described in detail. It should be understood, however, that the invention is not to be limited to the particular forms or methods disclosed, but to the contrary, the invention is to cover all modifications, equivalents and alternatives falling within the scope of the appended claims.

## Claims

1. A system for delivering a multiple component prosthetic valve into a biological annulus (92) within a patient's body, comprising:
an elongate tubular member (60) comprising a proximal end, a distal end (64) sized for introduction into a body lumen, and a lumen (62) extending between the proximal and distal ends (64);
a first annular prosthesis (12, 112, 112') implantable within the biological annulus (92), the annular prosthesis (12, 112, 112') being disposed within the tubular member adjacent the distal end in a contracted condition, the annular prosthesis (12, 112, 112') being expandable upon deployment from the tubular member within the biological annulus (92) to an enlarged condition; and
a second valve prosthesis (14, 114, 114', 214) securable to the annular prosthesis (12, 112, 112'), the valve prosthesis (14, 114, 114', 214) being disposed within the tubular member adjacent the annular prosthesis (12, 112, 112') in a contracted condition, the valve prosthesis (14, 114, 114', 214) being expandable upon deployment from the tubular member within the biological annulus (92) such that the valve prosthesis (14, 114, 114', 214) may be secured to the annular prosthesis (12, 112, 112'), **characterized in that** the system further comprises a plurality of elongate guide elements (96) including a first end attachable to tissue surrounding a biological annulus (92) and having sufficient length to extend from the biological annulus (92) to a percutaneous entry site, the guide elements (96) being sized for being received through respective portions of at least one of the annular prosthesis (12, 112, 112') and the valve prosthesis (14, 114, 114', 214).

2. The system of claim 1, further comprising a pusher member disposed within the tubular member (60), the pusher member adjacent at least one of the annular and valve prostheses, the pusher member being movable relative to the tubular member (60) for deploying at least one of the annular prosthesis (12) and the valve prosthesis (14).

3. The system of claim 1, wherein the guide elements (96) are sized such that the tubular member (60) may be advanced through a body lumen of a patient over the guide elements (96).

4. The system of claim 3, wherein the valve prosthesis (14) comprises a plurality of passages for receiving respective guide elements (96) therethrough such that the valve prosthesis (14) may be advanced over the guide elements (96).

5. The system of claim 3, wherein the annular prosthesis (12) comprises a plurality of passages for receiving respective guide elements (96) therethrough such that the annular prosthesis (12) may be advanced over the guide elements (96).

6. The system of claim 3, wherein each of the guide elements (196) comprises one or more connectors (197, 197') spaced apart from the first end for securing at least one of the annular prosthesis (112) and the valve prosthesis (114) relative to tissue to which the first end is attached.

7. The system of claim 6, wherein the one or more connectors (197') comprise a ratchet.

8. The system of claim 6, wherein the one or more connectors (197) comprise a ramped proximal surface and a blunt distal surface.

9. The system of claim 1, wherein the valve prosthesis (14) comprises a frame (32), the frame (32) being compressible into a folded condition for at least partially contracting the valve member (14) into the contracted condition, and wherein the frame (32) is formed from resilient material such that, when the valve prosthesis (14) is deployed from the tubular member (60), the frame (32) resiliently expands to direct the valve prosthesis (14) towards the enlarged condition.

10. The system of any preceding claim, wherein the valve prosthesis (114) comprises a flexible sleeve (132) comprising a plurality of leaflets (133), the sleeve (132) being contractible into the contracted condition.

11. The system of claim 10, wherein the sleeve (132) further comprises a plurality of pockets (134), the annular prosthesis (112) further comprising a plurality of posts (124) sized for being received in respective pockets (134) of the sleeve (132) for at least partially securing the valve prosthesis (114) relative to the annular prosthesis (112).

12. The system of claim 11, wherein the posts (124) and pockets (134) comprises cooperating connectors (197) for further securing the valve prosthesis (114) relative to the annular prosthesis (112).

13. The system of claim 1, wherein the first annular prosthesis (14) comprises an annular ring (18) and a sewing cuff (20) extending outwardly from the annular ring (18), the annular prosthesis (12) being contractible into a contracted condition for introduction through a body lumen and being expandable for deployment within a biological annulus (92).

14. The system of any preceding claim, further comprising one or more connectors (197) on at least one of the annular prosthesis (112) and the valve prosthesis (114) for securing the valve prosthesis (114) relative to the annular prosthesis (112).

15. The system of any preceding claim, wherein the valve prosthesis (14) comprises a frame (32), the frame (32) being foldable into a folded condition for at least partially contracting the valve prosthesis (14) into the contracted condition.

16. The system of any preceding claim, further comprising one or more fixturing elements for securing the valve prosthesis to the annular prosthesis.

17. The system of claim 16, wherein the one or more fixturing elements comprise one or more filaments including connectors thereon.

## Patentansprüche

1. System zum Einbringen einer mehrere Komponenten umfassenden Herzklappenprothese in einen biologischen Anulus (92) innerhalb eines Patientenkörpers, das Folgendes umfasst:
ein langgestrecktes schlauchförmiges Element (60), das ein proximales Ende, ein distales Ende (64) mit einer zur Einführung in ein Körperlumen geeigneten Größe und ein Lumen (62) aufweist, das sich zwischen dem proximalen und dem distalen Ende (64) erstreckt;
eine erste Ringprothese (12, 112, 112'), die im Inneren des biologischen Anulus (92) implantierbar ist, die Ringprothese (12, 112, 112') in dem schlauchförmigen Element benachbart zu dem distalen Ende in einem zusammengezogenen Zustand angeordnet ist, wobei die Ringprothese (12, 112, 112') nach dem Herausschieben aus dem schlauchförmigen Element in das Innere des biologischen Anulus (92) in einen ausgedehnten Zustand expandierbar ist; und
eine zweite Klappenprothese (14, 114, 114', 214), die an der Ringprothese (12, 112, 112') befestigbar ist, die Klappenprothese (14, 114, 114', 214) innerhalb des schlauchförmigen Elements in einem zusammengezogenen Zustand benachbart zu der Ringprothese (12, 112, 112') angeordnet ist, wobei die Klappenprothese (14, 114, 114', 214) nach dem Herausschieben aus dem schlauchförmigen Element in das Innere des biologischen Anulus (92) derart expandierbar ist; dass die Klappenprothese (14, 114, 114', 214) an der Ringprothese (12, 112, 112') festgemacht werden kann, **dadurch gekennzeichnet, dass** das System ferner mehrere längliche Führungselemente (96, 196) aufweist, die ein erstes Ende umfassen, das mit dem Gewebe, welches einen biologischen Anulus (92) umgibt, verknüpfbar ist und eine ausreichende Länge besitzen, um sich von dem biologischen Anulus (92) zu einer perkutanen Eintrittsstelle zu erstrecken, wobei die Führungselemente (96) so bemessen sind, dass sie durch die entsprechenden Abschnitte von mindestens einem aus der Ringprothese (12, 112, 112') und der Klappenprothese (14, 114, 114', 214) aufgenommen werden.

2. System nach Anspruch 1, das ferner ein Schiebeelement aufweist, das innerhalb des schlauchförmigen Elements (60) angeordnet ist, wobei das Schiebeelement zu mindestens einem von der Ring- oder der Klappenprothese benachbart ist, das Schiebeelement im Verhältnis zu dem schlauchförmigen Element (60) zum Einsetzen von mindestens einem von der Ringprothese (12) und der Klappenprothese (14) verschiebbar ist.

3. System nach Anspruch 1, wobei die Führungselemente (96) derart bemessen sind, dass das schlauchförmige Element (60) durch ein Körperlumen von einem Patienten über die Führungselemente (96) vorgeschoben werden kann.

4. System nach Anspruch 3, wobei die Klappenprothese (14) mehrere Durchgänge aufweist, durch die hindurch die entsprechenden Führungselemente (96) derart aufgenommen werden, dass die Klappenprothese (14) über die Führungselemente (96) vorgeschoben werden kann.

5. System nach Anspruch 3, wobei die Ringprothese (12) mehrere Durchgänge aufweist, durch die hindurch die entsprechenden Führungselemente (96) derart aufgenommen werden, dass die Ringprothese (12) über die Führungselemente (96) vorgeschoben werden kann.

6. System nach Anspruch 3, wobei jedes der Führungselemente (196) ein oder mehrere Verbindungselemente (197, 197') aufweist, die von dem ersten Ende räumlich getrennt sind, zum Befestigen von mindestens einer aus der Ringprothese (112) und der Klappenprothese (114) entsprechend an dem Gewebe, mit dem das erste Ende verknüpft ist.

7. System nach Anspruch 6, wobei das eine oder die mehreren Verbindungselemente (197') eine Sperrvorrichtung aufweisen.

8. System nach Anspruch 6, wobei das eine oder die mehreren Verbindungselemente (197) eine angeschrägte proximale Oberfläche und eine abgerundete distale Oberfläche aufweisen.

9. System nach Anspruch 1, wobei die Klappenprothese (14) einen Befestigungsrahmen (32) aufweist, der Befestigungsrahmen (32) in einen gefalteten Zustand zusammenpressbar ist, um das Klappenelement (14) mindestens teilweise in einen zusammengezogenen Zustand zusammenzuziehen, und wobei der Befestigungsrahmen (32) aus einem elastischen Material derart gebildet ist, dass, wenn die Klappenprothese (14) aus dem schlauchförmigen Element (60) herausgeschoben wird, der Befestigungsrahmen (32) elastisch expandiert, um die Klappenprothese (14) in Richtung des aufgeweiteten Zustandes auszudehnen.

10. System nach einem der vorangehenden Ansprüche, wobei die Klappenprothese (114) eine elastische Manschette (132) aufweist, die mehrere Klappensegel (133) umfasst, wobei die Manschette (132) in den zusammengezogenen Zustand zusammenziehbar ist.

11. System nach Anspruch 10, wobei die Manschette (132) ferner mehrere Aufnehmer (134) aufweist, die Ringprothese (112) ferner mehrere Ständer (124) aufweist, die derart bemessen sind, dass sie in die entsprechenden Aufnehmer (134) der Manschette (132) aufgenommen werden, um die Klappenprothese (114) entsprechend an der Ringprothese (112) mindestens teilweise zu befestigen.

12. System nach Anspruch 11, wobei die Ständer (124) und Aufnehmer (134) zusammenwirkende Verbindungselemente (197) für die weitere entsprechende Befestigung der Klappenprothese (114) an der Ringprothese (112) aufweisen.

13. System nach Anspruch 1, wobei die erste Ringprothese (14) einen Haltewulst (18) und einen Nahtring (20), der sich von dem Haltewulst nach außen erstreckt, aufweist, wobei die Ringprothese (12) zur Einführung durch ein Körperlumen in einen zusammengezogenen Zustand zusammenziehbar ist und zum Einsetzen innerhalb eines biologischen Anulus (92) expandierbar ist.

14. System nach einem der vorangehenden Ansprüche, das ferner ein oder mehrere Verbindungselemente (197) an mindestens einem von der Ringprothese (112) und der Klappenprothese (114) zur entsprechenden Befestigung der Klappenprothese (114) an der Ringprothese (112) aufweist.

15. System nach einem der vorangehenden Ansprüche, wobei die Klappenprothese (14) einen Befestigungsrahmen (32) aufweist, der Befestigungsrahmen (32) in einen gefalteten Zustand zusammenfaltbar ist, um die Klappenprothese (14) mindestens teilweise in den zusammengezogenen Zustand zusammenzuziehen.

16. System nach einem der vorangehenden Ansprüche, das ferner ein oder mehrere Befestigungselemente zum Festmachen der Klappenprothese an der Ringprothese aufweist.

17. System nach Anspruch 16, wobei das eine oder die mehreren Befestigungselemente einen oder mehrere Fäden, einschließlich der Verbindungselemente daran, aufweisen.

## Revendications

1. Système de mise en place d'une valve prothétique à composants multiples dans un anneau biologique (92) à l'intérieur du corps d'un patient qui comprend :
un élément tubulaire allongé (60) qui comprend une extrémité proximale, une extrémité distale (64) dimensionnée pour être introduite dans une lumière corporelle, et une lumière (62) s'étendant entre les extrémités proximale et distale (64) ;
une première prothèse annulaire (12, 112, 112') pouvant être implantée à l'intérieur de l'anneau biologique (92), la prothèse annulaire (12, 112, 112') étant disposée à l'intérieur de l'élément tubulaire adjacent à l'extrémité distale dans une condition contractée, la prothèse annulaire (12, 112, 112') pouvant passer lors du déploiement à partir de l'élément tubulaire à l'intérieur de l'anneau biologique (92) dans une condition dilatée ; et
une seconde prothèse valvulaire (14, 114, 114', 214) pouvant être fixée à la prothèse annulaire (12, 112, 112'), la prothèse valvulaire (14, 114, 114', 214) étant disposée à l'intérieur de l'élément tubulaire adjacent à la prothèse annulaire (12, 112, 112') dans une condition contractée, la prothèse valvulaire (14, 114, 114', 214) étant expansible lors du déploiement à partir de l'élément tubulaire à l'intérieur de l'anneau biologique (92) de telle sorte que la prothèse valvulaire (14, 114, 114', 214) peut être fixée à la prothèse annulaire (12, 112, 112'), **caractérisé en ce que** le système comprend en outre une pluralité d'éléments de guidage allongés (96, 196) comprenant une première extrémité pouvant être fixée à un tissu entourant un anneau biologique (92) et ayant une longueur suffisante pour s'étendre de l'anneau biologique (92) à un site d'entrée percutané, les éléments de guidage (96) étant dimensionnés pour être reçus dans des parties respectives d'au moins l'une de la prothèse annulaire (12, 112, 112') et de la prothèse valvulaire (14, 114, 114', 214).

2. Système selon la revendication 1, comprenant en outre un élément poussoir disposé à l'intérieur de l'élément tubulaire (60), l'élément poussoir étant adjacent à au moins l'une de la prothèse annulaire et de la prothèse valvulaire, l'élément poussoir étant amovible par rapport à l'élément tubulaire (60) pour déployer au moins l'une de la prothèse annulaire (12) et de la prothèse valvulaire (14).

3. Système selon la revendication 1, dans lequel les éléments de guidage (96) sont dimensionnés de manière à ce qu'il soit possible de faire progresser l'élément tubulaire (60) dans une lumière corporelle d'un patient sur les éléments de guidage (96).

4. Système selon la revendication 3, dans lequel la prothèse valvulaire (14) comprend une pluralité de passages destinés à recevoir des éléments de guidage respectifs (96) à l'intérieur de ceux-ci, de telle sorte qu'il est possible de faire progresser la prothèse valvulaire (14) sur les éléments de guidage (96).

5. Système selon la revendication 3, dans lequel la prothèse annulaire (12) comprend une pluralité de passages destinés à recevoir les éléments de guidage respectifs (96) à l'intérieur de ceux-ci, de telle sorte qu'il est possible de faire progresser la prothèse annulaire (12) sur les éléments de guidage (96).

6. Système selon la revendication 3, dans lequel chacun des éléments de guidage (196) comprend un ou plusieurs connecteurs (197, 197') espacés de la première extrémité pour fixer au moins l'une de la prothèse annulaire (112) et de la prothèse valvulaire (114) par rapport au tissu auquel la première extrémité est attachée.

7. Système selon la revendication 6, dans lequel le ou les connecteurs (197') comprennent un rochet.

8. Système selon la revendication 6, dans lequel le ou les connecteurs (197) comprennent une surface proximale inclinée et une surface distale émoussée.

9. Système selon la revendication 1, dans lequel la prothèse valvulaire (14) comprend un cadre (32), le cadre (32) étant compressible en une condition pliée pour contracter au moins partiellement l'élément de valve (14) dans la condition contractée, et le cadre (32) étant formé en un matériau résilient de telle sorte que, lorsque la prothèse valvulaire (14) est déployée à partir de l'élément tubulaire (60), le cadre (32) se déploie de manière résiliente pour diriger la prothèse valvulaire (14) dans la condition dilatée.

10. Système selon l'une quelconque des revendications précédentes, dans lequel la prothèse valvulaire (114) comprend un manchon flexible (132) comprenant une pluralité de feuillets (133), le manchon (132) pouvant être contracté dans la condition contractée.

11. Système selon la revendication 10, dans lequel le manchon (132) comprend en outre une pluralité de poches (134), la prothèse annulaire (112) comprenant en outre une pluralité de montants (124) dimensionnés pour être reçus dans les poches respectives (134) du manchon (132) pour fixer au moins partiellement la prothèse valvulaire (114) par rapport à la prothèse annulaire (112).

12. Système selon la revendication 11, dans lequel les montants (124) et les poches (134) comprennent des raccords coopérants (197) pour davantage fixer la prothèse valvulaire (114) par rapport à la prothèse annulaire (112).

13. Système selon la revendication 1, dans lequel la première prothèse annulaire (14) comprend une bague annulaire (18) et un manchon de suture (20) s'étendant vers l'extérieur à partir de la bague annulaire (18), la prothèse annulaire (12) pouvant être contractée dans une condition contractée pour être introduite dans une lumière corporelle et étant expansible pour être déployée à l'intérieur d'un anneau biologique (92).

14. Système selon l'une quelconque des revendications précédentes, qui comprend en outre un ou plusieurs raccords (197) sur au moins l'une de la prothèse annulaire (112) et de la prothèse valvulaire (114) pour fixer la prothèse valvulaire (114) par rapport à la prothèse annulaire (112).

15. Système selon l'une quelconque des revendications précédentes, dans lequel la prothèse valvulaire (14) comprend un cadre (32), le cadre (32) pouvant être plié en une condition pliée pour contracter au moins partiellement la prothèse valvulaire (14) dans la condition contractée.

16. Système selon l'une quelconque des revendications précédentes, comprenant en outre un ou plusieurs éléments de fixation destinés à fixer la prothèse valvulaire à la prothèse annulaire.

17. Système selon la revendication 16, dans lequel le ou les éléments de fixation comprennent un ou plusieurs filaments sur lesquels se trouvent des connecteurs.
